(19) Europäisches Patentamt
European Patent Office
Office européen des brevets

(11) Publication number: **0 257 616 B1**

(12) **EUROPEAN PATENT SPECIFICATION**

(45) Date of publication of patent specification: **28.10.92**

(51) Int. Cl.5: **C07D 211/90**, C07D 401/04, A61K 31/445, C07C 215/76, C07C 219/34, C07D 295/08

(21) Application number: **87112302.2**

(22) Date of filing: **25.08.87**

The file contains technical information submitted after the application was filed and not included in this specification

(54) **Dihydropyridine derivates and pharmaceutical composition thereof.**

(30) Priority: **27.08.86 JP 200849/86**
**23.10.86 JP 253077/86**

(43) Date of publication of application:
**02.03.88 Bulletin 88/09**

(45) Publication of the grant of the patent:
**28.10.92 Bulletin 92/44**

(84) Designated Contracting States:
**BE CH DE ES FR GB LI NL SE**

(56) References cited:
**EP-A- 0 026 317**
**EP-A- 0 088 903**
**EP-A- 0 097 821**
**EP-A- 0 191 448**

(73) Proprietor: **THE GREEN CROSS CORPORATION**
**3-3, Imabashi 1-chome Chuo-ku**
**Osaka-shi Osaka(JP)**

(72) Inventor: **Ashimori, Atsuyuki**
**B-41-203, Otokoyama Ishishiro 1-banchi**
**Yawata-shi, Kyoto(JP)**
Inventor: **Ono, Taizo / 22 Heights**
**Higashiyama, Nishi-iru**
**Karatohana-cho 561, Sanjo-Shirakawa-Sagaru**
**Higashiyama-ku, Kyoto-shi, Kyoto(JP)**
Inventor: **Inoue, Yoshihisa**
**15 Shion-so, Yoshida Naka-Adachi-cho 18**
**Sakyo-ku, Kyoto-shi, Kyoto(JP)**
Inventor: **Fukaya, Chikara**
**2-11-33-604, Kema-cho, Miyakojima-ku**
**Osaka-shi, Osaka(JP)**
Inventor: **Yokoyama, Kazumasa**
**2-7-2-201, Terauchi**
**Toyonaka-shi, Osaka(JP)**

(74) Representative: **von Kreisler, Alek, Dipl.-Chem. et al**
**Deichmannhaus am Hauptbahnhof**
**W-5000 Köln 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 257 616 B1

## Description

Field of the Invention

This invention relates to dihydropyridine derivatives and acid addition salts thereof which are novel and useful as pharmaceuticals and pharmaceutical composition thereof.

Background of the Invention

As the compounds similar to dihydropyridine derivatives of the present invention, there have been known, for example, nifedipine, nicardipine, etc. While these compounds have been known to be useful as an antihypertensive agent, a peripheral and cerebral vasodilating agent and a coronary artery-treating (angina pectoris-treating) agent, it has been demanded that dihydropyridine derivatives having still more excellent effect are presented.
EP-A-0 191 448 describes dihydropyridine derivatives which are useful for reacting congestive heart failure, hypertension or angina.

Summary of the Invention

The object of the present invention is to provide dihydropyridine derivatives and acid addition salts thereof which have still more excellent pharmacological activities.

Detailed Description of the Invention

This invention relates to a dihydropyridine derivative of a formula:

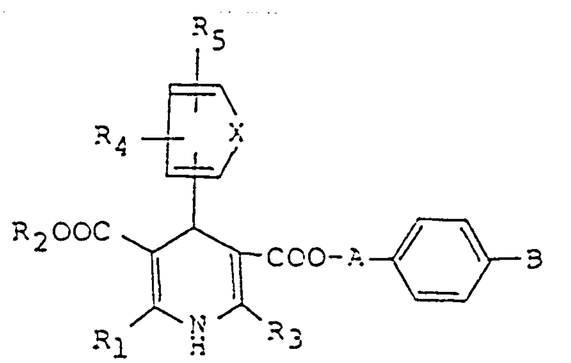

I

wherein $R_1$, $R_2$ and $R_3$ are the same or different, and each is $C_{1-6}$ alkyl, $C_{3-6}$ cycloalkyl or alkoxyalkyl; having $C_{3-7}$ in total; $R_4$ and $R_5$ are the same or different, and each is hydrogen atom, halogen, nitro, halogenated $C_{1-4}$ alkyl, $C_{1-6}$ alkylsulfonyl, halogenated $C_{1-3}$ alkoxy, $C_{1-6}$ alkylsulfinyl, $C_{1-6}$ alkyl, $C_{3-6}$ cycloalkyl, $C_{1-3}$ alkoxy, cyano, alkoxycarbonyl having $C_{2-4}$ in total or $C_{1-3}$ alkylthio (wherein both of $R_4$ and $R_5$ are not hydrogen atoms at the same time); X is a group represented by vinylene or azomethine; A is $C_{2-4}$ alkylene; and B is

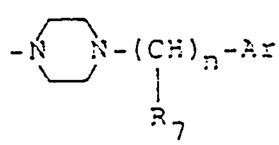

wherein $R_7$ is hydrogen atom, $C_{1-6}$ alkyl, $C_{3-4}$ cycloalkyl, phenyl $C_{1-3}$ allyl, phenyl, naphthyl or pyridyl, Ar is phenyl, naphthyl or pyridyl and n is an integer of 0 to 2) or an acid addition salt thereof.

These compounds [hereinafter referred to briefly as dihydropyridine derivatives (I)] have an excellent calcium blocking action (Ca-antagonist) an antihypertensive action, a platelet aggregation-inhibiting action, a phosphodiesterase-inhibiting action, and thus are useful as a medicine such as a coronary vasodilator, a

celebral hyperkinemic; antihypertensive, thrombosis-preventing or -treating agents, phophodiesterase-inhibitor.

The dihydropyridine derivative (I) of the present invention has a unique structure as compared with dihydropyridine compounds which have been so far concretely known, and has a specific activity due to the unique structure. Namely, the dihydropyridine derivatives (I) of the present invention and their acid addition salts are characterized remarkably in that they show a high organ and tissue-selectivity in vasodilating activities particularly and that they are very low in acute toxicity and thus highly safe.

In the above formula, the alkyl represented by $R_1$, $R_2$ and $R_3$ may be straight or branched and particularly a lower alkyl ($C_{1-6}$), such as methyl, ethyl, propyl, isopropyl, butyl, isobutyl, sec-butyl, t-butyl, pentyl, isopentyl, neopentyl, hexyl, etc; is preferable, and among others, $C_{1-4}$ alkyls are preferred. The alkyl may have at the terminus a lower cycloalkyl ($C_{3-6}$) (e.g. cyclopropylmethyl, cyclobutylethyl, cyclopentylmethyl). As the cycloalkyl, preferred is a lower ($C_{3-6}$) cycloalkyl, such as cyclopropyl, cyclobutyl, cyclopentyl and cyclohexyl. As the alkoxyalkyl, preferred is an alkoxyalkyl having 3 to 7 carbon atoms in total in the moiety, such as methoxyethyl, ethoxyethyl, propoxyethyl, isopropoxyethyl, butoxyethyl, methoxypropyl, 2-methoxy-1-methylethyl, 2-ethoxy-1-methylethyl.

The substituents represented by $R_4$ and $R_5$ may be the same or different, and they may be attached to the ring at any position. Preferably, they are attached to the ring at the 2- and/or 3-positions relative to the position at which the dihydropyridine ring bond to the ring. The halogen represented by $R_4$ and $R_5$ include fluorine atom, chlorine atom, bromine atom and iodine atom, among which fluorine and chlorine atoms are particularly preferable. As the alkyl and cycloalkyl represented by $R_4$ and $R_5$, preferred are the alkyls and cycloalkyls which are mentioned above as the examples for $R_1$ to $R_3$. As the alkoxy and alkylthio, preferred are alkoxys and alkylthios having a lower alkyl ($C_{1-3}$), which are exemplified by methoxy, ethoxy, propoxy and isopropoxy, and methylthio, ethylthio, propylthio and isopropylthio, respectively. As the alkoxycarbonyl, there can be mentioned those having 2 to 4 carbon atoms such as methoxycarbonyl and ethoxycarbonyl. As the halogens of the halogenides, there are mentioned such halogens as mentioned above. The halogenated alkyls include those in which a part of the hydrogen atoms are halogenated [e.g. $(CF_3)_2CHCH_2$-, $CF_3CH_2$] and those in which all the hydrogen atoms are halogenated (e.g. trifluoromethyl). The alkyl moiety of the halogenated alkyl has preferably 1 to 4 carbon atoms. The halogenated alkoxys also include those in which a part of the hydrogen atoms are halogenated and those in which all the hydrogen atoms are halogenated. The alkyl moiety of the halogenated alkoxy preferably contains 1 to 3 carbon atoms. As the alkyl in the alkylsulfonyl and the alkylsulfinyl, there may be mentioned the alkyls which are mentioned above as the examples for $R_1$ to $R_3$.

As $R_4$ and $R_5$, particularly preferred are cyano and halogenated alkyls (particularly, trifluoromethyl).

As the alkyl and cycloalkyl represented by $R_7$, there can be mentioned the alkyls and the cycloalkyls which are mentioned above as the examples for $R_1$ to $R_3$. As the aralkyl, there can be mentioned phenyl $C_{1-3}$-alkyl such as benzyl, $\alpha$-phenylethyl, $\beta$-phenylethyl and $\gamma$-phenylpropyl, and as the aryl, phenyl and naphthyl can be mentioned. The aromatic ring of them may have the same or different substituents at the optional position of the ring. As the substituent on the aromatic ring, there may be mentioned, for example, those which are mentioned above as the examples for $R_4$ and $R_5$. The pyridyl includes 2-pyridyl, 3-pyridyl and 4-pyridyl, which may be substituted by the groups mentioned as the examples for $R_4$ and $R_5$.

As the alkylene represented by A, preferable are those having 2 to 4 carbon atoms and they may be straight or branched. They are exemplified by ethylene, trimethylene, tetramethylene, 1,2-dimethylethylene.

The aryl and pyridyl represented by Ar include, for example, those which are mentioned above as the examples for $R_7$, and they may have the same substituents as the aryl and pyridyl represented by $R_7$.

The ring represented by the formula

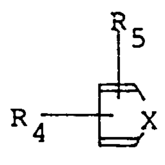

as the substituent at the 4-position of dihydropyridine means a benzene ring in case where X is vinylene (-CH=CH-), and a pyridine in case where X is azomethine (-CH=N-), and the ring may bind to the 4-position of the dihydropyridine at the optional position of itself.

The substituents represented by $R_4$ and $R_5$ may be attached to the ring at any of the orto-, metha- and para-positions relative to the carbon atom binding to the 4-position of the dihydropyridine, and are attached

preferably to the ortho- and/or metha-positions of the ring.

The dihydropyridine derivatives (I) can be produced by reacting a compound containing the optional constituent moiety of the said dihydropyridine derivatives (I) and the compounds containing the remaining constituent moiety thereof by a per se known means, particularly by subjecting them to dehydration ring-closure reaction. For example, the objective compounds can be produced in the following manner.

Production Method:

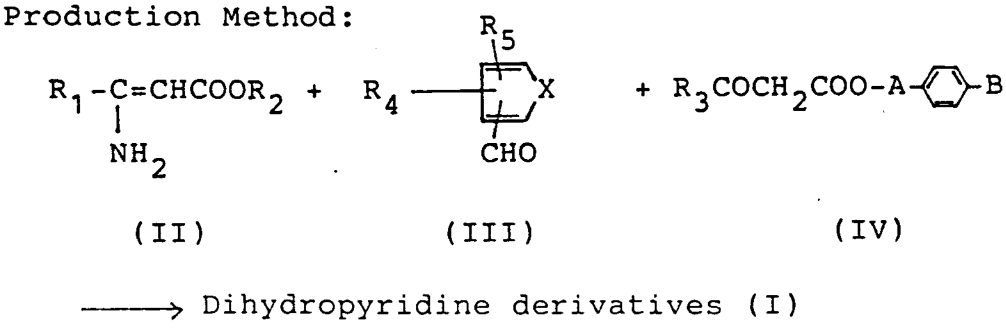


(II) (III) (IV)

In the scheme, $R_1$, $R_2$, $R_3$, $R_4$, $R_5$, X, A and B have the same meanings as defined above.

The reaction of compounds (II), (III) and (IV) are usually carried out at temperatures ranging from about 20 ° C to about 160 ° C, preferably from about 30 ° C to about 130 ° C. As the solvent, any solvent can be used so long as it is inert to the reaction. Suitable solvents include, for example, alkanols such as methanol, ethanol, propanol, isopropanol, butanol, sec-butanol, ethers such as ethyl ether, dioxane, tetrahydrofuran, ethylene glycol monomethyl ether, ethylene glycol dimethyl ether, acetic acid, pyridine, N,N-dimethylformamide, dimethylsulfoxide, acetonitrile. As for the amount of compounds (II), (III) and (IV) to be used, based on 1 mol of one of the three compounds, 1 to 1.5 mol of the other two compounds are usually used. The reaction is usually completed in about 1 to 30 hours.

Dihydropyridine derivatives (I) can be produced by the method or a method similar to the one disclosed in European Patent Publication No. 94159.

The main starting compound (IV) of the present invention can be synthesized, for example, by the following route.

③

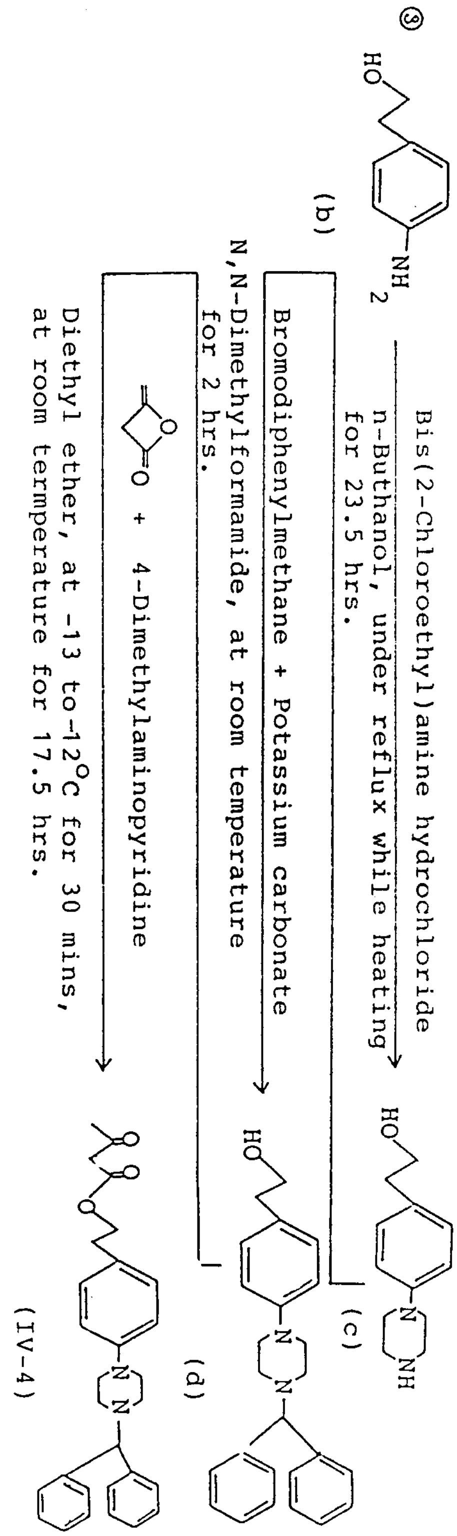


(b) (c) (d) (IV-4)

The compound (IV) including the compound (IV-4) and the compound (c) and (d) are used as starting material in the production of the dihydropyridine derivatives (I); these compounds can be summarized by the following formula

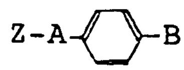

(A)

wherein Z is hydroxyl or the group represented by the formula

$R_3$-CO-$CH_2$-COO-

$R_3$ is $C_{1\sim6}$ alkyl, $C_{3\sim6}$ cycloalkyl or alkoxyalkyl having $C_{3\sim7}$ in total; A is alkylene; and B is a group of the formula

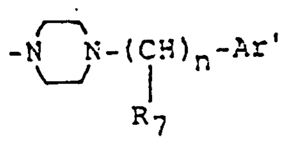

(wherein $R_7$ is hydrogen atom, $C_{1\sim6}$ alkyl, $C_{3\sim6}$ cycloalkyl, phenyl $C_{1\sim3}$ alkyl, phenyl, naphthyl or pyridyl, Ar' is hydrogen atom, phenyl, naphthyl or pyridyl and n is an integer of 0 to 2) [hereinafter referred to briefly as compound (A)]

The compound (A) is generally produced by the methods described as follows.

Method (i):

The compound wherein Z is hydroxyl, B is

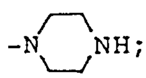

namely the compound (A-2); and the compound wherein Z is hydroxyl and B is

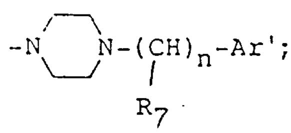

namely the compound (A-3)

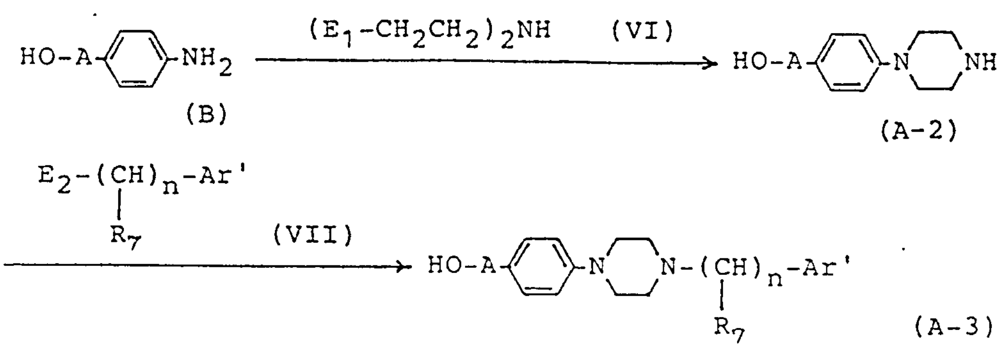


(B)

(A-2)

(A-3)

wherein A, Ar', $R_7$ and n are as defined above, and $E_1$ and $E_2$ respectively show halogen atom.

The compound (A-2) is obtained by reacting compound (B) with compound (VI). The present reaction can be carried out through reflux under heating for 20 to 30 hours in the presence of an inert solvent (e.g. n-butanol, sec-butanol, propanol).

Then, the compound (A-3) of the present invention can be obtained by reacting compound (A-2) with compound (VII). This reaction is carried out in the presence of an inert solvent (e.g. N,N-dimethylformamide (DMF), dimethylsulfoxide, diglyme, ethylene glycol monomethyl ether) for 1-5 hours at 10-120°C. It is favorable that the reaction be conducted in the presence of alkali, such as potassium carbonate.

Method (ii):

The compound wherein Z is $R_3$-CO-$CH_2$-COO-, namely the compound (A-4)

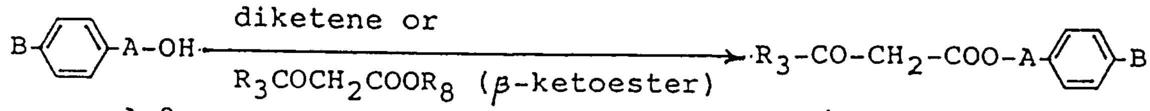


A-2 or A-3)

(VIII)

(A-4)

wherein $R_8$ represents a lower alkyl (usually $C_{1-4}$) and all the other symbols are as defined above.

That is, the compound (A-4) is produced by reacting compound (A-2) or (A-3) with diketene or compound (VIII).

The reaction of compound (A-2) or (A-3) with diketene is conducted by heating a mixture of the both compounds at about 40°C to about 130°C without any solvent or in a solvent inert to the reaction; or, it can also be carried out in the presence of a catalyst, such as p-dimethylaminopyridine, at about -20°C to about 40°C in a solvent inert to the reaction (e.g. dimethyl ether, tetrahydrofuran, dimethoxyethane).

Also, the compound (A-4) of the present invention can be produced by reacting compound (A-2) or (A-3) with compound (VIII). This reaction is conducted in the presence of a base (e.g. sodium methoxide, sodium ethoxide, potassium t-butoxide, sodium hydride, sodium amide, metal sodium) at about 20°C to about 100°C in an inert solvent or without solvent.

The novel dihydropyridine derivatives (I) thus produced can be purified to an optional extent by a suitable known separation and purification means such as concentration, extraction, chromatography, reprecipitation, or recrystallization.

Besides, since the dihydropyridine derivatives (I) have basic groups, they can be converted into acid addition salts thereof by a known means. As for such salts, there is no limitation so long as they are pharmacologically acceptable nontoxic salts, as are exemplified by salts with inorganic acids (e.g. hydrochloride, hydrobromide, phosphate, sulfate) and those with organic acids (e.g. acetate, succinate, maleate, fumarate, malate, tartrate).

The dihydropyridine derivatives (I) and acid addition salts thereof are extremely low in toxicity and have an antihypertensive action, a peripheral vasodilating action, a coronary artery-dilating action, a cerebral vasodilating action and other actions which are potent and lasting in mammals (e.g. mouse, rat, rabbit, dog, cat, man). Thus, they are useful as a medicine for prophylaxis and treatment of circulatory diseases such as hypertension, ischemic cardiac diseases (angina pectoris, myocardial infarction), cerebral and peripheral circulation disturbances (cerebral infarction, temporary cerebral ischemic spasm) and the like.

In particular, the dihydropyridine derivatives (I) and acid addition salts thereof are more excellent in their potency and duration of pharmacological actions as compared with previously known dihydropyridine derivatives (e.g. nifedipine, nicardipine). Thus, for example, when they are used as a medicine for prophylaxis or treatment of hypertension, they give a stable antihypertensive action by a fewer times' dosage (once or twice a day).

When the dihydropyridine derivatives (I) and their acid addition salts are used as medicines mentioned above, they can be mixed with pharmaceutically required ingredients such as pharmacologically acceptable, appropriate additives (e.g. carrier, excipient, diluent) to give a pharmaceutical composition in a form such as powders, granules, tablets, capsules or injection, which can be orally or parenterally administered. The dihydropyridine derivatives (I) and their acid addition salts are incorporated in the above-mentioned pharmaceutical compositions in a pharmaceutically effective amount. While the dosage varies depending upon the administration route, severity of the diseases, the body weight or age of the patient, or the like, when they are orally administered to an adult patient suffering from hypertension, for example, they can be administered in an amount of 0.05 to 20 mg/kg body weight/day, preferably 0.1 to 4 mg/kg body weight/day in one to several divided doses a day.

Experimental Example

The results of the pharmacological tests showing the effectivity of the dihydropyridine derivatives (I) and acid addition salts thereof of the present invention are given below.

(1) Antihypertensive Action

The experiments were conducted with the use of male rats (three to five rats per a group) 10 to 11 weeks old and suffering from spontaneous hypertention. For blood pressure determination, systolic pressure was measured without anesthesia by an indirect tail-cuff method using a sphygmomanometer (PE-300, Narco Bio-System).

The test compounds were respectively suspended in 10% HCO-60 [general name: polyoxyethylene hardened castor oil, manufactured by Nikko Chemicals Corporation (Japan)] and the suspensions were orally administered in an amount of 25 mg/kg. After the administration, the blood pressure was measured with the lapse of time. The maximum ratio of decrease in the blood pressure (%) of the said compounds ranged from 4 to 33%. The period of time during which the decreased blood pressure value took to recover to the level of 50% of the blood pressure value before the administration was within the range of 5 to 17 hours.

(2) Acute Texicity

The $LD_{50}$ was estimated with the use of male mice (3 to 5 mice per a group) at the age of 10 to 11 weeks, weighing 14-16 g. The results were at least more than 1100 mg/Kg and those of most test compounds were more than 1400 mg/Kg.

Accordingly, the compounds which the present invention provides are significantly lower in their acute toxicity than known compounds and thus safer.

The present invention is explained in further detail by illustrating below working examples, which are not to be construed to be limitative of the present invention.

In $^1$H-NMR measurement, unless otherwise specified, $CDCl_3$ was used.

Example 1

(1) 2-(p-Piperazinophenyl)ethanol

In a 200 ml-pear-shaped type flask were put 2-(p-aminophenyl)ethanol (10.153 g, 74.0 mmol) and bis(2-chloroethyl)amine hydrochloride (6.605 g, 37.0 mmol), whereto n-butanol (66 ml) was added. With a Dimroth condenser equipped, the mixture was refluxed under heating for 23.5 hours. After the reaction mixture was cooled to the neighborhood of room temperature, it was added to water (218 ml) in a 500 ml-beaker. Under ice-cooling, a 15% aqueous solution of sodium hydroxide was added to adjust to pH 10 to 11, and the mixture was extracted with chloroform (five times, 300 ml each time). The chloroform layer was washed with brine (twice, 150 ml each time) and dried. The residue (13.485 g) obtained by distillation under reduced pressure was subjected to column chromatography [silica gel, chloroform-methanol (1:1)] for separation and purification to give 5.996 g of the above-captioned piperazine compound (Yield: 79%).

$$IR\nu_{max}^{KBr}cm^{-1}: 3300(OH)$$

$^1$H-NMRδ:
7.15-6.95 (2H),
6.95-6.7 (2H),
3.77 (2H, t, J = 6Hz),
3.2-2.8 (8H),
2.75 (2H, t, J = 6Hz),
2.10 (2H, s)

(2) 2-[p-(4-Benzhydrylpiperazino)phenyl]ethanol

The product of (1) (the piperazine compound) (5.996 g, 29.1 mmol) was put in a 100 ml-three-necked flask, which was equipped with a Dimroth condenser on the central mouth and septum rubbers on the side mouths. In the flask, DMF (33 ml) was added to dissolve the content, and potassium carbonate (8.034 g, 58.1 mmol) and bromodiphenylmethane (7.543 g, 30.5 mmol) were added. The mixture was stirred at room temperature for 2 hours. The reaction mixture was added to water (80 ml) in a 200 ml-conical flask, and the mixture was extracted with diethyl ether (four times, 100 ml each time). The ether layer was washed with brine (twice, 40 ml each time), dried and distilled off under reduced pressure. The residue (10.616 g) was subjected to column chromatography [silica gel, ethyl acetate-n-hexane (1:1)] for separation and purification to give 6.188 g of the above-captioned benzhydrylpiperazine compound (Yield: 57%).

$$IR\nu_{max}^{CHCl3} cm^{-1}: 3600(OH)$$

$^1$H-NMRδ:
7.55-6.9 (12H),
6.9-6.55 (2H),
4.23 (1H, s),
3.72 (2H, t, J=6Hz),
3.3-2.95 (4H),
2.95-2.35 (6H),
1.71 (1H, s)

(3) 2-[p-(4-Benzhydrylpiperazino)phenyl]ethyl acetoacetate

The product of (2) (the benzhydrylpiperazine compound) (5.970 g, 16.0 mmol) was put in a 200 ml-three-necked flask, which was equipped with an air condenser on the central mouth, a thermometer on one of the side mouths and a septum rubber on the other mouth. In the flask, diethyl ether (50 ml) was added to dissolve the content. After the mixture was cooled to -13 to -12°C (with ice-sodium chloride), diketene (1.531 g, 18.2 mmol) and DMAP (7 mg, 0.057 mmol) were added thereto. The mixture was stirred at the same temperature for 30 minutes and then at room termperature for 17.5 hours. The reaction mixture was ice-cooled, washed by adding a 0.1% aqueous solution of sodium hydroxide (65 ml), and separated into a water layer and a diethyl ether layer. The water layer was extracted with ether (three times, 120 ml each time). The ether extract was combined with the said diethyl ether layer and it was washed with a 0.1% aqueous solution of sodium hydroxide (twice, 65 ml each time) and then with water (twice, 50 ml each time), dried, and was distilled off under reduced pressure to give 7.364 g of the above-captioned acetoacetic acid ester stoichiometrically.

$$IR\nu_{max}^{neat} cm^{-1}: 1740(C=O), 1720(C=O),$$

$^1$H-NMRδ:
7.6-6.9 (12H),
6.9-6.65 (2H),
4.29 (2H, t, J=6.5Hz),
4.24 (1H, s),
3.39 (2H, s),
3.35-3.0 (4H),
2.85 (2H, t, J=6.5Hz),
2.7-2.4 (4H),
2.18 (3H, s)

(4) 2-[p-(4-benzhydrylpiperazino)phenyl]ethyl methyl 2,6-dimethyl-4-(4-cyano-2-pyridyl)-1,4-dihydropyridine-3,5-dicarboxylate (compound 5-1) and its monohydrochloride (compound 5-2)

In a 50 ml-pear-shaped flask were put 4-cyano-2-pyridinealdehyde (684 mg, 5.18 mmol), the objective compound of (3) (2.363 g, 5.18 mmol) and methyl 3-aminocrotonate (614 mg, 5.18 mmol), whereto

isopropanol (7 ml) was added. With a Dimroth condenser equipped on the flask, the mixture was stirred at 40 to 45°C for 26 hours. The reaction solvent was distilled off under reduced pressure, and the obtained residue (3.539 g) was subjected to column chromatography [silica gel, ethyl acetate-n-hexane (3:1)] and [silica gel, ethyl acetate-n-hexane (2:1)] for separation to give a crude product. The crude product was recrystallized from chloroform-methanol to give 1.649 g of the compound 5-1 (Yield: 48%). m.p. (what was recrystallized from chloroform-methanol): 218-220°C

$$IR\nu_{max}^{KBr}cm^{-1}: 2225(CN),\ 1700(C=O),\ 1665(C=O)$$

$^1$H-NMRδ:
8.65-8.45 (1H),
7.6-6.65 (17H),
5.16 (1H, s),
4.4-4.0 (3H),
3.59 (3H, s),
3.3-2.95 (4H),
2.95-2.45 (6H),
2.25, 2.22 (each 3H, s)

The compound 5-1 (1.092 g, 1.64 mmol) was put in a 100 ml-three-necked flask, which was equipped with an air condenser on the central mouth and septum rubbers on the side mouths. In the flask, methylene chloride (21 ml) was added to dissolve the content, and a dioxane solution of hydrogen chloride (1.20 N, 1.363 ml) was added. The mixture was stirred at room temperature for 5 hours. After the reaction solvent was distilled off under reduced pressure, ethanol (15 ml) was added to the residue, and the mixture was distilled off under reduced pressure to give about 1.15 g of the compound 5-2.

$$IR\nu_{max}^{KBr}cm^{-1}: 2225(CN),\ 1680(C=O)$$

$^1$H-NMR$\delta_{DMSO\text{-}d6 + CDCl3(2:1.5)}$:
8.74 (1H, s),
8.57 (1H, d, J = 5Hz),
7.55-7.1 (12H),
7.1-6.6 (4H),
5.05 (1H, s),
4.35-3.95 (2H),
3.75-2.6 (14H),
2.25, 2.23 (each 3H, s)

(5) <u>2-[p-(4-Benzhydrylpiperazino)phenyl] ethyl methyl 2,6-dimethyl-4-(2-trifluoromethyl-3-pyridyl)-1,4-dihydropyridine-3,5-dicarboxylate (compound 6-1) and its monofumarate (compound 6-2)</u>

2-Trifluoromethyl-3-pyridinealdehyde (961 mg, 5.49 mmol), the objective compound of (2) (the acetoacetic acid ester compound, 2.279 g, 4.99 mmol) and methyl 3-amino-crotonate (592 mg, 4.99 mmol) were added in a 50 ml-pear-shaped flask, whereto isopropanol (9 ml) was added. With a Dimroth condenser equipped on the flask, the mixture was stirred at 45 to 48°C for 27.5 hours. The reaction solvent was distilled off under reduced pressure and the residue (3.714 g) was subjected to column chromatography [silica gel, chloroform-methanol (98.5:1.5)] and [silica gel, ethyl acetate-n-hexane (3:2)] for separation and purification to give 512 mg of the compound 6-1 (Yield: 13%).

$$IR\nu_{max}^{KBr}cm^{-1}: 1700(C=O)$$

$^1$H-NMRδ:
8.55-8.35 (1H),

7.95-7.75 (1H),
7.55-7.0 (11H),
7.0-6.6 (4H),
5.86 (1H, s),
5.59 (1H, s),
4.45-3.95 (3H),
3.57 (3H, s),
3.3-2.95 (4H),
2.95-2.6 (6H),
2.28, 2.24 (each 3H, s)

The compound 6-1 (473 mg, 0.665 mmol) was put in a 50 ml-pear-shaped flask, whereto ethanol (20 ml) was added to dissolve the content, and fumaric acid (77 mg, 0.665 mmol) was added. With an air condenser equipped on the flask, the mixture was stirred at room temperature for an hour. The reaction solvent was distilled off under reduced pressure to give about 550 mg of the compound.

$$IR\nu^{KBr}_{max} cm^{-1}: 3350(CO\underline{H}), \quad 2500(N^{+}H^{-}O_2C-) \quad 1690(C=O)$$

$^1$H-NMR$\delta_{DMSO\text{-}d6 + CDCl3(2:1.5)}$:
8.67 (1H, s),
8.5-8.3 (1H),
8.0-7.8 (1H),
7.6-7.05 (11H),
7.05-6.65 (4H),
6.64 (2H, s),
5.45 (1H, s),
4.4-3.85 (3H),
3.48 (3H, s),
3.3-2.9 (4H),
2.9-2.3 (6H),
2.26, 2.20 (each 3H, s),

(6) <u>2-[4-(4-Benzhydrylpiperazino)phenyl]ethyl methyl 2,6-dimethyl-4-(3-nitrophenyl)-1,4-dihydropyridine-3,5-dicarboxylate (compound 7-1) and its monohydrochloride (compound 7-2)</u>

In a 100 ml pear-shaped flask were put 3-nitrobenzaldehyde (1.144 g, 7.57 mmol), the objective compound of (3) (3.464 g, 7.59 mmol) and methyl 3-aminocrotonate (873 mg, 7.58 mmol), whereto isopropanol (12 ml) was added. With a Dimroth condenser equipped on the flask, the mixture was refluxed for 16 hours. The reaction solvent was distilled off under reduced pressure, and the obtained residue was subjected to column chromatography [silica gel, chloroform-methanol (45:1)] and [silica gel, ethyl acetate-n-hexane (2:3)] for separation to give a crude product. The crude product was purified by HPLC to give 2.503 g of the compound 7-1 (Yield: 48%).

$$IR\nu^{KBr}_{max} cm^{-1}: 1680(C=O), \quad 1520(NO_2),$$

$^1$H-NMR$\delta$:
8.06 (1H, t, J = 2Hz),
7.97 (1H, ddd, J = 8;2;1Hz),
7.1-7.6 (12H),
7.03 (2H, d, J = 8.6Hz),
6.80 (2H, d, J = 8.6Hz),

6.02 (1H, s),
5.07 (1H, s),
4.26 (1H, s),
4.22 (2H, t, J = 7Hz),
3.64 (3H, s),
3.15 (4H, dd, J = 5;4.7Hz),
2.81 (2H, t, J = 7Hz),
2.55 (4H, dd, J = 5;4.7Hz),
2.33, 2.28 (each 3H, s)

The compound 7-1 (2.124 g; 3.16 mmol) was put in a 200 ml pear-shaped flask, which was equipped with a septum rubber. In the flask, methylene chloride (100 ml) was added to dissolve the content, and a dioxane solution of hydrogen chloride (1.20 N, 2.64 ml) was added. The mixture was stirred at room temperature for 30 minutes. The reaction solvent was evaporated off under reduced pressure to give about 2.22 g of the compound 7-2.

$$IR\nu_{max}^{KBr}cm^{-1}: 2450(\rightarrow N^{+}H^{-}Cl), 1680(C=O), 1520(NO_2),$$

$^1$H-NMR$\delta_{DMSO-D6}$:
9.17 (1H, s),
8.1-7.9, 7.5-7.3 (14H),
7.08 (2H, d, J = 8Hz),
6.85 (2H, d, J = 8Hz),
5.73 (1H, d, J = 9Hz),
4.97 (1H, s),
4.14 (2H, t, J = 6Hz),
3.8-3.55 (7H),
3.23 (4H, brs),
2.77 (2H, $t_1$ J = 6Hz),
2.30, 2.26 (each 3H, s)

Example 2

(1) 3-[4-(4-benzhydrylpiperazino)phenyl]propanol

Using 3-(p-aminophenyl)propanol instead of 2-(p-aminophenyl)ethanol, there was obtained the objective compound in the same manner as in Example 1(1)(2).

Nature of the objective compound: colorless oil

$$IR\nu_{max}^{CHCl3}cm^{-1}: 3600(OH)$$

$^1$H-NMR$\delta$:
7.5-7.05 (1OH), 7.05-6.85 (2H), 6.85-6.6 (2H),
4.19 (1H, s), 3.55 (2H, t, J = 6Hz), 3.25-2.95 (4H),
2.75-2.35 (6H), 2.05-1.45 (2H)

(2) 3-[4-(4-benzhydrylpiperazino)phenyl]propyl acetoacetate

Using the objective compound of Example 2(1), there was obtained the objective compound in the same manner as in

Example 3(2).

$$IR\nu_{max}^{film}cm^{-1}: 1740(C=O), 1720(C=O)$$

$^1$H-NMRδ:
7.6-7.1 (1OH), 7.1-6.9 (2H), 6.9-6.65 (2H),
4.27 (1H, s), 4.12 (2H, t, J=6Hz), 3.42 (2H, s),
3.35-3.0 (4H), 2.8-2.4 (6H), 2.26 (3H, s), 2.1-1.6 (2H)

## Claims

**Claims for the following Contracting States : BE, CH, DE, FR, GB, LI, NL, SE**

**1.** A dihydropyridine derivative of a formula:

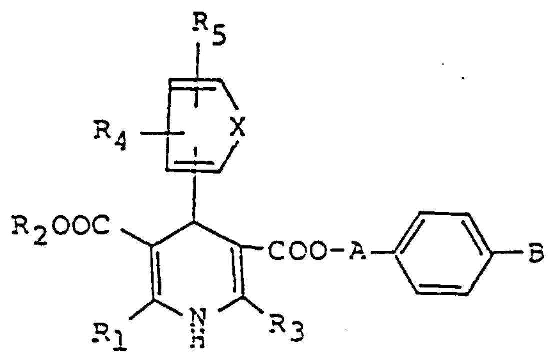

(I)

wherein $R_1$, $R_2$ and $R_3$ are the same or different, and each is $C_{1-6}$ alkyl, $C_{3-6}$ cycloalkyl or alkoxyalkyl having $C_{3-7}$ in total; $R_4$ and $R_5$ are the same or different, and each is hydrogen atom, halogen, nitro, halogenated $C_{1-4}$ alkyl, $C_{1-6}$ alkylsulfonyl, halogenated $C_{1-3}$ alkoxy, $C_{1-6}$ alkylsulfinyl, $C_{1-6}$ alkyl, $C_{3-6}$ cycloalkyl, $C_{1-3}$ alkoxy, cyano, alkoxycarbony having $C_{2-4}$ in total or $C_{1-3}$ alkylthio (wherein both of $R_4$ and $R_5$ are not hydrogen atoms at the same time); X is a group represented by vinylene or azomethine; A is $C_{2-4}$ alkylene; and B is

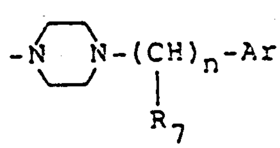

wherein $R_7$ is hydrogen atom, $C_{1-6}$ alkyl, $C_{3-6}$ cycloalkyl, phenyl $C_{1-3}$ alkyl, phenyl, naphthyl or pyridyl, Ar is phenyl, naphthyl or pyridyl and n is an integer of 0 to 2), or an acid addition salt thereof.

**2.** A dihydropyridine derivative as claimed in Claim 1, wherein A is ethylene, or an acid addition salt thereof.

**3.** A dihydropyridine derivative as claimed in Claim 1, wherein X is azomethine or an acid addition salt thereof.

**4.** A dihydropyridine derivative as claimed in Claim 1, wherein X is vinylene or an acid addition salt thereof.

**5.** A dihydropyridine derivative as claimed in Claim 1, wherein Ar is phenyl or an acid addition salt thereof.

**6.** A dihydropyridine derivative as claimed in Claim 1,

wherein both $R_7$ and Ar are phenyl optionally having substituents and n is 1 or an acid addition salt thereof.

**7.** A dihydropyridine derivative as claimed in Claim 1,
wherein Ar is phenyl optionally having substituents, X is vinylene, A is ethylene and n is O, or an acid addition salt thereof.

**8.** A dihydropyridine derivative as claimed in Claim 1,
wherein both $R_7$ and Ar are phenyl, X is vinylene, and n is 1, or an acid addition salt thereof.

**9.** A dihydropyridine derivative as claimed in Claim 1,
wherein X is azomethine, $R_4$ or $R_5$ is trifluoromethyl or cyano, A is ethylene, and B is

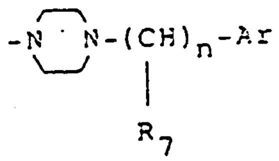

(wherein both $R_7$ and Ar are phenyl and n is 1), or an acid addition salt thereof.

**10.** A dihydropyridine derivative as claimed in Claim 1,
wherein X is vinylene, $R_4$ or $R_5$ is nitro, A is ethylene, and B is

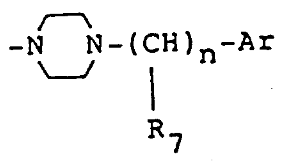

(wherein both $R_7$ and Ar are phenyl and n is 1), or an acid addition salt thereof.

**11.** A pharmaceutical composition which comprises an effective amount of at least one species selected from among the dihydropyridine derivatives as claimed in Claims 1 to 10 and acid addition salts thereof, and pharmaceutically acceptable additives.

**12.** A compound of the formula:

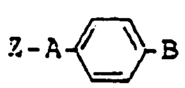

wherein Z is hydroxyl or a group represented by the formula:

$R_3$-CO-$CH_2$-COO-,

$R_3$ is $C_{1-6}$ alkyl, $C_{3-6}$ cycloalkyl or alkoxyalkyl having $C_{3-7}$ in total; A is $C_{2-4}$ alkylene; and B is a group of the formula

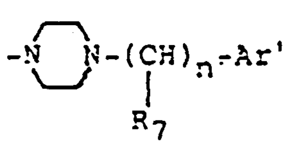

(wherein $R_7$ is hydrogen atom, $C_{1-6}$ alkyl, $C_{3-6}$ cycloalkyl, phenyl $C_{1-3}$ alkyl, phenyl, naphthyl or pyridyl, Ar' is hydrogen atom, phenyl, naphthyl or pyridyl and n is an integer of 0 to 2)

**Claims for the following Contracting State : ES**

1. Process for the production of a dihydropyridine derivative of a formula:

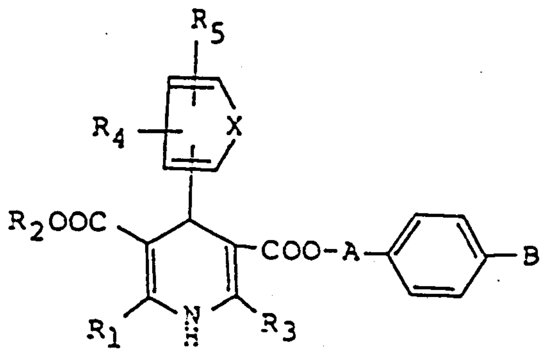

wherein $R_1$, $R_2$ and $R_3$ are the same or different, and each is $C_{1-6}$ alkyl, $C_{3-6}$ cycloalkyl or alkoxyalkyl having $C_{3-7}$ in total, $R_4$ and $R_5$ are the same or different, and each is hydrogen atom, halogen, nitro, halogenated $C_{1-4}$ alkyl, $C_{1-6}$ alkylsulfonyl, halogenated $C_{1-3}$ alkoxy, $C_{1-6}$ alkylsulfinyl, $C_{1-6}$ alkyl, $C_{3-6}$ cycloalkyl, $C_{1-3}$ alkoxy, cyano, alkoxycarbonyl having $C_{2-4}$ in total or $C_{1-3}$ alkylthio (wherein both of $R_4$ and $R_5$ are not hydrogen atoms at the same time); X is a group represented by vinylene or azomethine; A is $C_{2-4}$ alkylene; and B is

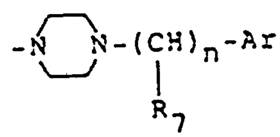

wherein $R_7$ is hydrogen atom, $C_{1-6}$ alkyl, $C_{3-6}$ cycloalkyl, phenyl $C_{1-3}$ alkyl, phenyl, naphthyl or pyridyl, Ar is phenyl, naphthyl or pyridyl and n is an integer of 0 to 2) or an acid addition salt thereof, comprising subjecting the compounds of the formulae

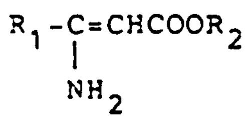

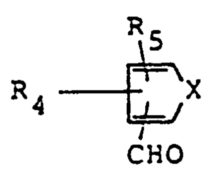

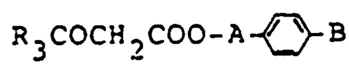

(II) (III) (IV)

wherein $R_1$, $R_2$, $R_3$, $R_4$, $R_5$, X, A and B have the same meanings as defined above, by dehydration ring closure reaction.

2. A process as claimed in Claim 1,
wherein A is ethylene, or an acid addition salt thereof.

3. A process as claimed in Claim 1,
wherein X is azomethine or an acid addition salt thereof.

4. A process as claimed in Claim 1,

wherein X is vinylene or an acid addition salt thereof.

**5.** A process as claimed in Claim 1, wherein Ar is phenyl or an acid addition salt thereof.

**6.** A process as claimed in Claim 1, wherein both $R_7$ and Ar are phenyl optionally having substituents and n is 1 or an acid addition salt thereof.

**7.** A process as claimed in Claim 1, wherein Ar is phenyl optionally having substituents, X is vinylene, A is ethylene and n is O, or an acid addition salt thereof.

**8.** A process as claimed in Claim 1, wherein both $R_7$ and Ar are phenyl, X is vinylene, and n is 1, or an acid addition salt thereof.

**9.** A process as claimed in Claim 1, wherein X is azomethine, $R_4$ or $R_5$ is trifluoromethyl or cyano, A is ethylene, and B is

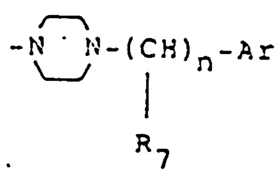

(wherein both $R_7$ and Ar are phenyl and n is 1), or an acid addition salt thereof.

**10.** A process as claimed in Claim 1, wherein X is vinylene, $R_4$ or $R_5$ is nitro, A is ethylene, and B is

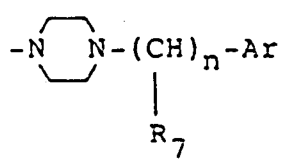

(wherein both $R_7$ and Ar are phenyl and n is 1), or an acid addition salt thereof.

**11.** Use of a dihydropyridine derivative produced by the process of claims 1 to 10 for the production of a pharmaceutical composition which comprises an effective amount of at least one species selected from among said dihydropyridine derivatives and acid addition salts thereof, and pharmaceutically acceptable additives.

**12.** A process for the production of a compound of the formula:

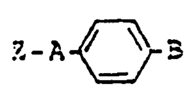

wherein Z is hydroxyl or a group represented by the formula:

$R_3$-CO-$CH_2$-COO-,

$R_3$ is $C_{1-6}$ alkyl, $C_{3-6}$ cycloalkyl or alkoxyalkyl having $C_{3-7}$ in total; A is $C_{2-4}$ alkylene; and B is a group of the formula

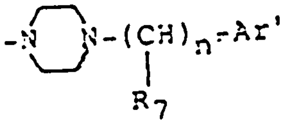

(wherein $R_7$ is hydrogen atom, $C_{1-6}$ alkyl, $C_{3-6}$ cycloalkyl, phenyl $C_{1-3}$ alkyl, phenyl, naphthyl or pyridyl, Ar' is hydrogen atom, phenyl, naphthyl or pyridyl and n is an integer of 0 to 2), comprising reacting the compound of the formula

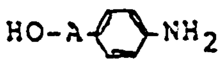

(B)

wherein A is as defined above with a compound of the formula

$(E_1\text{-}CH_2CH_2)_2NH$ (VI)

wherein E and $E_1$ respectively is halogen atom,
to give a compound of the formula

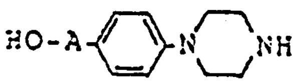

(A-2)

respectively, wherein the symbols are as defined above; and optionally reacting the compound (A-2) with a compound of the formula

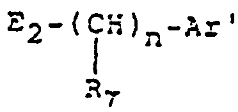

(VII)

wherein Ar', n and $R_7$ are as defined above and $E_2$ is halogen atom to give the compound of the formula

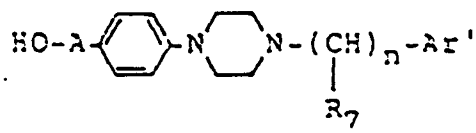

(A-3)

wherein A, Ar', n and $R_7$ are as defined above;
and optionally reacting the compounds (A-2) or (A-3) with a compound of the formula

$R_3COCH_2COOR_8$ (VIII)

or a diketene wherein $R_3$ is as defined above and $R_8$ represents a $C_{1-4}$ alkyl to give a compound of the formula

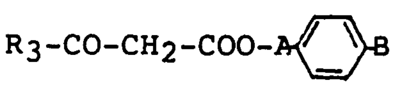

(A-4)

wherein the symbols are as defined above.

**Patentansprüche**

**Patentansprüche für folgende Vertragsstaaten : BE, CH, DE, FR, GB, LI, NL, SE**

1. Dihydropyridin-Derivat der Formel

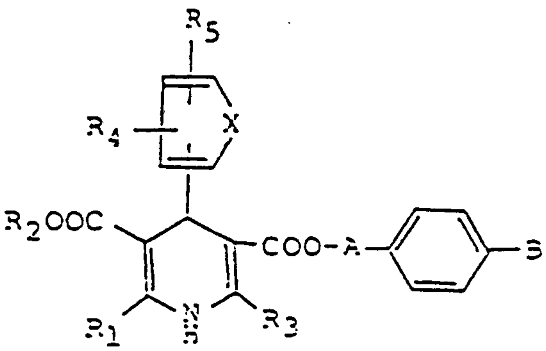

(I)

worin $R_1$, $R_2$ und $R_3$ gleich oder verschieden sind, und jedes $C_1$-$C_6$-Alkyl, $C_3$-$C_6$-Cycloalkyl oder Alkoxyalkyl mit insgesamt $C_3$-$C_7$ ist; $R_4$ und $R_5$ gleich oder verschieden sind, und jedes ein Wasserstoff-Atom, Halogen, Nitro, halogeniertes $C_1$-$C_4$-Alkyl, $C_1$-$C_6$-Alkylsulfonyl, halogeniertes $C_1$-$C_3$-Alkoxy, $C_1$-$C_6$-Alkylsulfinyl, $C_1$-$C_6$-Alkyl, $C_3$-$C_6$-Cycloalkyl, $C_1$-$C_3$-Alkoxy, Cyan, Alkoxycarbonyl mit insgesamt $C_2$-$C_4$ oder $C_1$-$C_3$-Alkylthio (worin die beiden $R_4$ und $R_5$ nicht gleichzeitig Wasserstoff-Atome sind); X eine Gruppe ist, die Vinylen oder Azomethin bedeutet; A $C_2$-$C_4$-Alkylen ist; und B

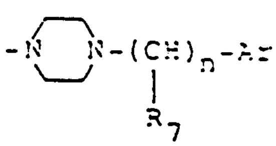

ist (worin $R_7$ ein Wasserstoff-Atom, $C_1$-$C_6$-Alkyl, $C_3$-$C_6$-Cycloalkyl, Phenyl-$C_1$-$C_3$-alkyl, Phenyl, Naphthyl oder Pyridyl ist, Ar Phenyl, Naphthyl oder Pyridyl ist, und n eine ganze Zahl von 0 bis 2 ist), oder ein Säureadditionssalz desselben.

2. Dihydropyridin-Derivat nach Anspruch 1, worin A Ethylen ist, oder ein Säureadditionssalz desselben.

3. Dihydropyridin-Derivat nach Anspruch 1, worin X Azomethin ist, oder ein Säureadditionssalz desselben.

4. Dihydropyridin-Derivat nach Anspruch 1, worin X Vinylen ist, oder ein Säureadditionssalz desselben.

5. Dihydropyridin-Derivat nach Anspruch 1, worin Ar Phenyl ist, oder ein Säureadditionssalz desselben.

6. Dihydropyridin-Derivat nach Anspruch 1, worin $R_7$ und Ar beide Phenyl sind, gegebenenfalls mit Substituenten, und n 1 ist, oder ein Säureadditionssalz desselben.

7. Dihydropyridin-Derivat nach Anspruch 1, worin Ar Phenyl ist, gegebenenfalls mit Substituenten, X Vinylen ist, A Ethylen ist, und n 0 ist, oder ein Säureadditionssalz desselben.

8. Dihydropyridin-Derivat nach Anspruch 1, worin $R_7$ und Ar beide Phenyl sind, X Vinylen ist, und n 1 ist, oder ein Säureadditionssalz desselben.

9. Dihydropyridin-Derivat nach Anspruch 1, worin X Azomethin ist, $R_4$ oder $R_5$ Trifluormethyl oder Cyan ist, A Ethylen ist, und B

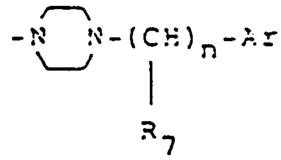

ist (worin $R_7$ und Ar beide Phenyl sind und n 1 ist), oder ein Säureadditionssalz desselben.

**10.** Dihydropyridin-Derivat nach Anspruch 1, worin X Vinylen ist, $R_4$ oder $R_5$ Nitro ist, A Ethylen ist, und B

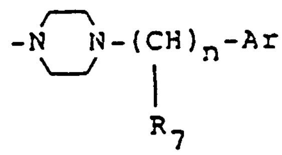

ist (worin $R_7$ und Ar beide Phenyl sind und n 1 ist), oder ein Säureadditionssalz desselben.

**11.** Pharmazeutische Zusammensetzung, umfassend eine wirksame Menge wenigstens einer Species, ausgewählt aus den Dihydropyridin-Derivaten nach den Ansprüchen 1 bis 10 und dessen Säureadditionssalzen, sowie pharmazeutisch annehmbare Zusätze.

**12.** Verbindung der Formel:

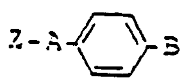

worin Z Hydroxyl ist oder eine Gruppe, dargestellt durch die Formel

$R_3$-CO-$CH_2$-COO-,

$R_3$ ist $C_1$-$C_6$-Alkyl, $C_3$-$C_6$-Cycloalkyl oder Alkoxyalkyl mit insgesamt $C_3$-$C_7$; A $C_2$-$C_4$-Alkylen ist; und B eine Gruppe der Formel

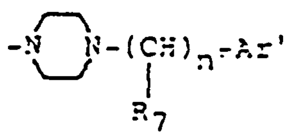

ist (worin $R_7$ ein Wasserstoff-Atom, $C_1$-$C_6$-Alkyl, $C_3$-$C_6$-Cycloalkyl, Phenyl-$C_1$-$C_3$-alkyl, Phenyl, Naphthyl oder Pyridyl ist, Ar' ein Wasserstoff-Atom, Phenyl, Naphthyl oder Pyridyl ist, und n eine ganze Zahl von 0 bis 2 ist).

**Patentansprüche für folgenden Vertragsstaat : ES**

**1.** Verfahren zur Herstellung eines Dihydropyridin-Derivats der Formel

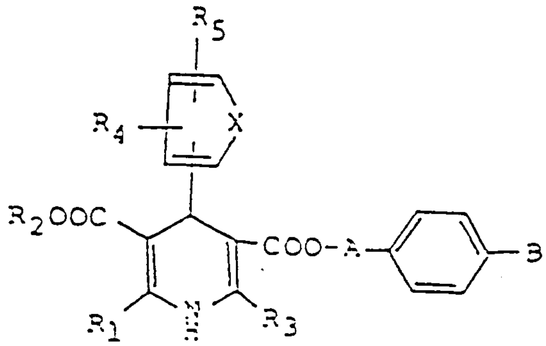

worin $R_1$, $R_2$ und $R_3$ gleich oder verschieden sind, und jedes $C_1$-$C_6$-Alkyl, $C_3$-$C_6$-Cycloalkyl oder Alkoxyalkyl mit insgesamt $C_3$-$C_7$ ist, $R_4$ und $R_5$ gleich oder verschieden sind, und jedes ein Wasserstoff-Atom, Halogen, Nitro, halogeniertes $C_1$-$C_4$-Alkyl, $C_1$-$C_6$-Alkylsulfonyl, halogeniertes $C_1$-$C_3$-Alkoxy, $C_1$-$C_6$-Alkylsulfinyl, $C_1$-$C_6$-Alkyl, $C_3$-$C_6$-Cycloalkyl, $C_1$-$C_3$-Alkoxy, cyan, Alkoxycarbonyl mit insgesamt $C_2$-$C_4$ oder $C_1$-$C_3$-Alkylthio (worin die beiden $R_4$ und $R_5$ nicht gleichzeitig Wasserstoff-Atome sind); X eine Gruppe ist, die Vinylen oder Azomethin bedeutet; A $C_2$-$C_4$-Alkylen ist; und B

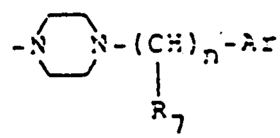

ist (worin $R_7$ ein Wasserstoff-Atom, $C_1$-$C_6$-Alkyl, $C_3$-$C_6$-Cycloalkyl, Phenyl-$C_1$-$C_3$-alkyl, Phenyl, Naphthyl oder Pyridyl ist, Ar Phenyl, Naphthyl oder Pyridyl ist, und n eine ganze Zahl von 0 bis 2 ist), oder ein Säureadditionssalz desselben, umfassend das Unterwerfen der Verbindungen der Formeln

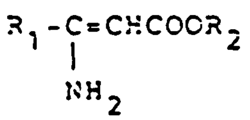

(II)

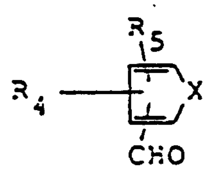

(III)

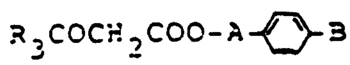

(IV)

worin $R_1$, $R_2$, $R_3$, $R_4$, $R_5$, X, A und B die gleichen Bedeutungen wie oben definiert haben, einer Ringschlußreaktion durch Dehydratisierung.

2. Verfahren nach Anspruch 1, wobei A Ethylen ist, oder ein Säureadditionssalz desselben.

3. Verfahren nach Anspruch 1, wobei X Azomethin ist, oder ein Säureadditionssalz desselben.

4. Verfahren nach Anspruch 1, wobei X Vinylen ist, oder ein Säureadditionssalz desselben.

5. Verfahren nach Anspruch 1, wobei Ar Phenyl ist, oder ein Säureadditionssalz desselben.

6. Verfahren nach Anspruch 1, wobei $R_7$ und Ar beide Phenyl sind, gegebenenfalls mit Substituenten, und n 1 ist, oder ein Säureadditionssalz desselben.

7. Verfahren nach Anspruch 1, wobei Ar Phenyl ist, gegebenenfalls mit Substituenten, X Vinylen ist, A Ethylen ist, und n 0 ist, oder ein Säureadditionssalz desselben.

8. Verfahren nach Anspruch 1, wobei $R_7$ und Ar beide Phenyl sind, X Vinylen ist, und n 1 ist, oder ein Säureadditionssalz desselben.

9. Verfahren nach Anspruch 1, wobei X Azomethin ist, $R_4$ oder $R_5$ Trifluormethyl oder Cyan ist, A Ethylen ist, und B

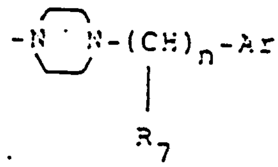

ist (worin $R_7$ und Ar beide Phenyl sind und n 1 ist), oder ein Säureadditionssalz desselben.

10. Verfahren nach Anspruch 1, wobei X Vinylen ist, $R_4$ oder $R_5$ Nitro ist, A Ethylen ist, und B

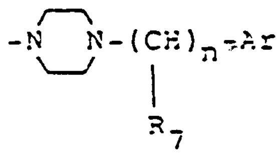

ist (worin $R_7$ und Ar beide Phenyl sind und n 1 ist), oder ein Säureadditionssalz desselben.

11. Verwendung eines Dihydropyridin-Derivats, hergestellt mit Hilfe des Verfahrens nach den Ansprüchen 1 bis 10, zur Herstellung einer pharmazeutischen Zusammensetzung, umfassend eine wirksame Menge wenigstens einer Species, ausgewählt aus diesen Dihydropyridin-Derivaten und deren Säureadditionssalzen, und pharmazeutisch annehmbare Zusätze.

12. Verfahren zur Herstellung einer Verbindung der Formel:

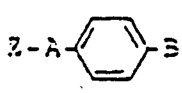

worin Z Hydroxyl ist oder eine Gruppe, dargestellt durch die Formel

$R_3$-CO-$CH_2$-COO-,

$R_3$ ist $C_1$-$C_6$-Alkyl, $C_3$-$C_6$-Cycloalkyl oder Alkoxyalkyl mit insgesamt $C_3$-$C_7$; A $C_2$-$C_4$-Alkylen ist; und B eine Gruppe der Formel

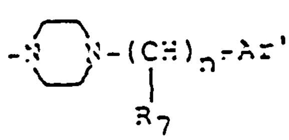

ist (worin $R_7$ ein Wasserstoff-Atom, $C_1$-$C_6$-Alkyl, $C_3$-$C_6$-Cycloalkyl, Phenyl-$C_1$-$C_3$-alkyl, Phenyl, Naphthyl oder Pyridyl ist, Ar' ein Wasserstoff-Atom, Phenyl, Naphthyl oder Pyridyl ist, und n eine ganze Zahl

von 0 bis 2 ist), umfassend das Umsetzen einer Verbindung der Formel

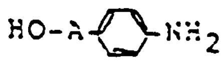

(B)

worin A wie vorstehend definiert ist, mit einer Verbindung der Formel

$(E_1\text{-}CH_2CH_2)_2NH$ (VI)

worin E bzw. $E_1$ ein Halogen-Atom ist, um eine Verbindung der Formel

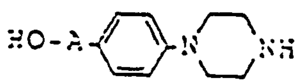

(A-2)

zu ergeben, worin die Symbole wie vorstehend definiert sind; und gegebenenfalls Umsetzen der Verbindung (A-2) mit einer Verbindung der Formel

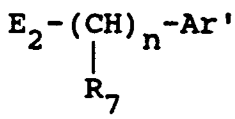

(VII)

worin Ar', n und $R_7$ wie vorstehend definiert sind und $E_2$ ein Halogen-Atom ist, um eine Verbindung der Formel

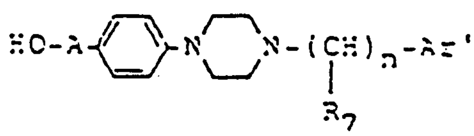

(A-3)

zu ergeben, worin A, Ar', n und $R_7$ wie vorstehend definiert sind;
und gegebenenfalls Umsetzen der Verbindungen (A-2) oder (A-3) mit einer Verbindung der Formel

$R_3COCH_2COOR_8$ (VIII)

oder einem Diketen, worin $R_3$ wie vorstehend definiert ist und $R_8$ $C_1$-$C_4$-Alkyl bedeutet, um eine Verbindung der Formel

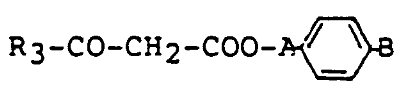

(A-4)

zu ergeben, worin die Symbole wie vorstehend definiert sind.

## Revendications

**Revendications pour les Etats contractants suivants : BE, CH, DE, FR, GB, LI, NL, SE**

**1.** Dérivé de dihydropyridine de formule

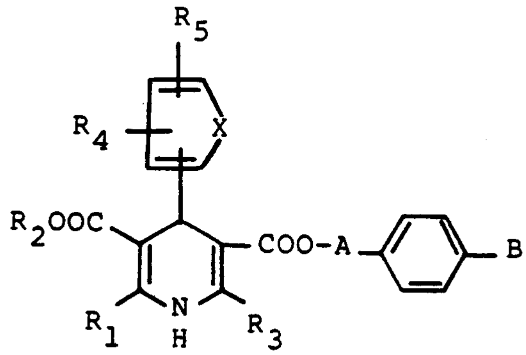

. (I)

dans laquelle $R_1$, $R_2$ et $R_3$ sont les mêmes ou différents, et chacun est un alkyle $C_{1-6}$, cycloalkyle $C_{3-6}$ ou alcoxyalkyle ayant $C_{3-7}$ en tout ; $R_4$ et $R_5$ sont identiques ou différents, et chacun est un atome d'hydrogène, halogène, nitro, alkyle $C_{1-4}$ halogéné, alkylsulfonyle $C_{1-6}$, alcoxy $C_{1-3}$ halogéné, alkylsulfinyle $C_{1-6}$, alkyle $C_{1-6}$, cycloalkyle $C_{3-6}$, alcoxy $C_{1-3}$, cyano, alcoxycarbonyle ayant $C_{2-4}$ en tout ou alkylthio $C_{1-3}$ (dans lequel $R_4$ et $R_5$ ne représentent pas l'hydrogène en même temps) ; X est un groupe représenté par le vinylène ou l'azométhine ; A est un alkylène $C_{2-4}$ ; et B est

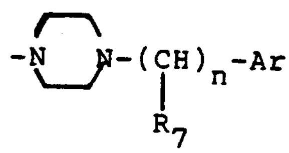

dans lequel $R_7$ est un atome d'hydrogène, alkyle $C_{1-6}$, cycloalkyle $C_{3-6}$, phényl(alkyle $C_{1-3}$), phényle, naphtyle ou pyridyle, Ar est un phényle, naphtyle ou pyridyle et n est un entier de 0 à 2), ou un de ses sels d'addition d'acide.

**2.** Dérivé de dihydropyridine selon la revendication 1, dans lequel A est l'éthylène ou un de ses sels d'addition d'acide.

**3.** Dérivé de dihydropyridine selon la revendication 1, dans lequel X est l'azométhine ou un de ses sels d'addition d'acide.

**4.** Dérivé de dihydropyridine selon la revendication 1, dans lequel X est le vinylène ou un de ses sels d'addition d'acide.

**5.** Dérivé de dihydropyridine selon la revendication 1, dans lequel Ar est le phényle ou un de ses sels d'addition d'acide.

**6.** Dérivé de dihydropyridine selon la revendication 1, dans lequel $R_7$ et Ar représentent tous les deux le phényle éventuellement substitué, et n est 1 ou un de ses sels d'addition d'acide.

**7.** Dérivé de dihydropyridine selon la revendication 1, dans lequel Ar est un phényle qui a éventuellement des substituants, X est le vinylène, A est l'éthylène et n est 0, ou un de ses sels d'addition d'acide.

**8.** Dérivé de dihydropyridine selon la revendication 1, dans lequel $R_7$ et Ar représentent le phényle, X est un vinylène, et n est 1 ou un de ses sels d'addition d'acide.

**9.** Dérivé de dihydropyridine selon la revendication 1, dans lequel X est l'azométhine, $R_4$ ou $R_5$ représentent un trifluorométhyle ou cyano, A est l'éthylène, et B est

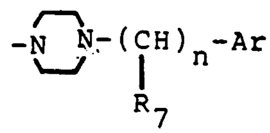

(dans lequel $R_7$ et Ar représentent tous les deux le phényle et n est 1), ou un de ses sels d'addition d'acide.

10. Dérivé de dihydropyridine selon la revendication 1, dans lequel X est le vinylène, $R_4$ ou $R_5$ est un nitro, A est l'éthylène et B est

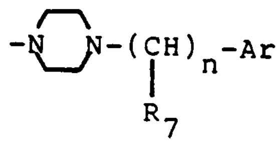

(dans lequel $R_7$ et Ar représentent tous les deux le phényle et n est 1), ou un de leurs sels d'addition d'acide.

11. Composition pharmaceutique qui comprend une quantité efficace d'au moins une espèce choisie parmi les dérivés de dihydropyridine selon les revendications 1 à 10 et leurs sels d'addition d'acide pharmaceutiquement acceptables.

12. Composé de formule :

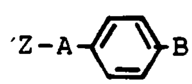

dans laquelle Z est un hydroxyle ou un groupe représenté par la formule :

$R_3$-CO-$CH_2$-COO-,

$R_3$ est un alkyle $C_{1-6}$, cycloalkyle $C_{3-6}$ ou alcoxyalkyle ayant $C_{3-7}$ en tout, A est un alkylène $C_{2-4}$ ; et B est un groupe de formule

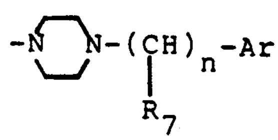

(dans laquelle $R_7$ est un atome d'hydrogène, alkyle $C_{1-6}$, cycloalkyle $C_{3-6}$, phényl(alkyle $C_{1-3}$), phényle, naphtyle ou pyridyle et n est un entier de 0 à 2).

**Revendications pour l'Etat contractant suivant : ES**

1. Procédé de production d'un dérivé de dihydropyridine de formule :

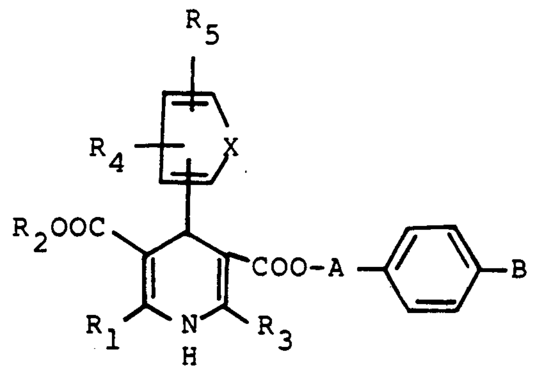

(I)

dans laquelle $R_1$, $R_2$ et $R_3$ sont les mêmes ou différents, et chacun est un alkyle $C_{1-6}$, cycloalkyle $C_{3-6}$ ou alcoxyalkyle ayant $C_{3-7}$ en tout ; $R_4$ et $R_5$ sont identiques ou différents, et chacun est un atome d'hydrogène, halogène, nitro, alkyle $C_{1-4}$ halogéné, alkylsulfonyle $C_{1-6}$, alcoxy $C_{1-3}$ halogéné, alkylsulfinyle $C_{1-6}$, alkyle $C_{1-6}$, cycloalkyle $C_{3-6}$, alcoxy $C_{1-3}$, cyano, alcoxycarbonyle ayant $C_{2-4}$ en tout ou alkylthio $C_{1-3}$ (dans lequel $R_4$ et $R_5$ ne représentent pas l'hydrogène en même temps) ; X est un groupe représenté par le vinylène ou l'azométhine ; A est un alkylène $C_{2-4}$ ; et B est

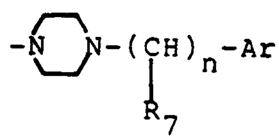

dans lequel $R_7$ est un atome d'hydrogène, alkyle $C_{1-6}$, cycloalkyle $C_{3-6}$, phényl(alkyle $C_{1-3}$), phényle, naphtyle ou pyridyle, Ar est un phényle, naphtyle ou pyridyle et n est un entier de 0 à 2), ou un de ses sels d'addition d'acide, qui comprend de soumettre les composés de formules

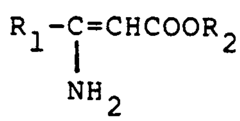

(II)

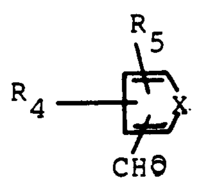

(III)

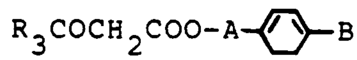

(IV)

dans lesquelles $R_1$, $R_2$, $R_3$, $R_4$, $R_5$, X, A et B ont les mêmes significations que celles données ci-dessus, à une réaction de fermeture de cycle par déshydratation.

2. Procédé selon la revendication 1, dans lequel A est l'éthylène ou un de ses sels d'addition d'acide.

3. Procédé selon la revendication 1, dans lequel X est l'azométhine ou un de ses sels d'addition d'acide.

4. Procédé selon la revendication 1, dans lequel X est le vinylène ou un de ses sels d'addition d'acide.

5. Procédé selon le revendication 1, dans lequel Ar est le phényle ou un de ses sels d'addition d'acide.

6. Procédé selon la revendication 1, dans lequel $R_7$ et Ar représentent tous les deux le phényle éventuellement substitué, et n est 1 ou un de ses sels d'addition d'acide.

7. Procédé selon la revendication 1, dans lequel Ar est un phényle qui a éventuellement des substituants, X est le vinylène, A est l'éthylène et n est 0, ou un de ses sels d'addition d'acide.

**8.** Procédé selon la revendication 1, dans lequel $R_7$ et Ar représentent le phényle, X est un vinylène, et n est 1 ou un de ses sels d'addition d'acide.

**9.** Procédé selon la revendication 1, dans lequel X est l'azométhine, $R_4$ ou $R_5$ représentent un trifluorométhyle ou cyano, A est l'éthylène, et B est

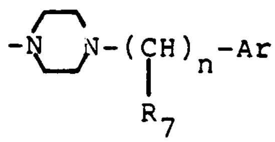

(dans lequel $R_7$ et Ar représentent tous les deux le phényle et n est 1), ou un de ses sels d'addition d'acide.

**10.** Procédé selon la revendication 1, dans lequel X est le vinylène, $R_4$ ou $R_5$ est un nitro, A est l'éthylène et B est

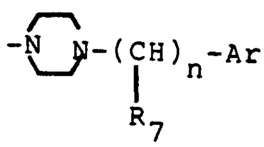

(dans lequel $R_7$ et Ar représentent tous les deux le phényle et n est 1), ou un de leurs sels d'addition d'acide.

**11.** Utilisation d'un dérivé de dihydropyridine produit par le procédé des revendications 1 à 10 pour produire une composition pharmaceutique qui comprend une quantité efficace d'au moins une espèce choisie parmi lesdits dérivés de dihydropyridine et leurs sels d'addition d'acide, et des additifs pharmaceutiquement acceptables.

**12.** Procédé de production d'un composé de formule :

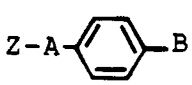

dans laquelle Z est un hydroxyle ou un groupe représenté par la formule :

$R_3$-CO-$CH_2$-COO-,

$R_3$ est un alkyle $C_{1-6}$, cycloalkyle $C_{3-6}$ ou alcoxyalkyle ayant $C_{3-7}$ en tout ; A est un alkylène $C_{2-4}$ ; et B est un groupe de formule

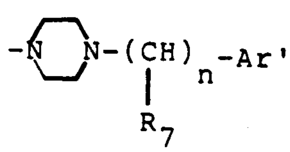

(dans laquelle $R_7$ est un atome d'hydrogène, alkyle $C_{1-6}$, cycloalkyle $C_{3-6}$, phényl(alkyle $C_{1-3}$), phényle, naphtyle ou pyridyle, Ar' est un atome d'hydrogène, phényle, naphtyle ou pyridyle et n est un entier de 0 à 2), qui comprend la mise en réaction du composé de formule

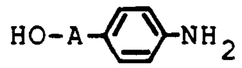

(B)

dans laquelle A est tel que défini ci-dessus avec un composé de formule

$(E_1\text{-}CH_2CH_2)_2NH$ (VI)

dans laquelle E et $E_1$ représentent respectivement un atome d'halogène, pour donner un composé de formule

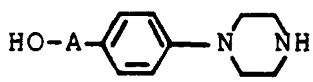

(A-2)

respectivement, dans laquelle les symboles sont tels que définis ci-dessus ; et éventuellement en faisant réagir le composé (A-2) avec un composé de formule

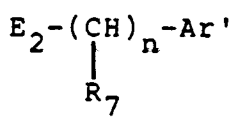

(VII)

dans laquelle Ar', n et $R_7$ sont tels que définis ci-dessus et $E_2$ est un atome d'halogène pour donner le composé de formule

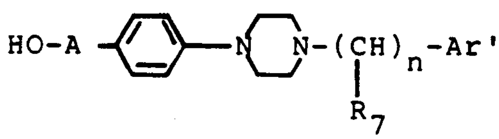

(A-3)

dans laquelle A, Ar', n et $R_7$ sont tels que définis ci-dessus ;
et en variante en faisant réagir les composés (A-2) ou (A-3) avec un composé de formule

$R_3COCH_2COOR_8$ (VIII)

ou un dicétène dans laquelle $R_3$ est tel que défini ci-dessus et $R_8$ représente un alkyle $C_{1-4}$ pour donner un composé de formule

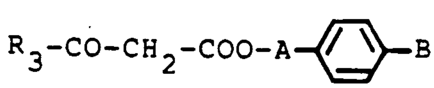

(A-4)

dans laquelle les symboles sont tels que définis ci-dessus.